# EUROPEAN PATENT SPECIFICATION

(11) **EP 1 345 608 B1**
(45) Date of publication and mention of the grant of the patent: **04.04.2007**
(21) Application number: 01995843.8
(22) Date of filing: 21.11.2001
(51) Int. Cl.: A61K 31/496, A61P 15/02, A61P 15/10

(54) **SELECTIVE DOPAMINE D4 RECEPTOR AGONISTS FOR TREATING SEXUAL DYSFUNCTION**
SELEKTIVE DOPAMIN-D4-REZEPTORAGONISTEN ZUR BEHANDLUNG VON SEXUELLEN DYSFUNKTIONEN
AGONISTES SELECTIFS DES RECEPTEURS DE LA DOPAMINE D4 POUR TRAITER DES DYSFONCTIONNEMENTS SEXUELS

(30) Priority: 22.11.2000 US 718311; 07.11.2001 US 985974
(43) Date of publication of application: 24.09.2003
(73) Proprietor: ABBOTT LABORATORIES, Abbott Park, IL 60064-6050 (US)
(72) Inventor: BRIONI, Jorge, D., Vernon Hills, IL 60061 (US); KOLASA, Teodozyj, Lake Villa, IL 60046 (US); HSIEH, Gin, C., Long Grove, IL 60047 (US); DONNELLY-ROBERTS, Diane, L., Lake Villa, IL 60046 (US)
(74) Representative: Modiano, Micaela Nadia
(86) International application number: PCT/US2001/043139
(87) International publication number: WO 2002/041894

(56) References cited:
- EP-A- 0 982 030
- WO-A-00/35457
- WO-A-96/04250
- US-A- 5 945 117
- DATABASE MEDLINE [Online] US NATIONAL LIBRARY OF MEDICINE (NLM), BETHESDA, MD, US; issn 0028-3843, 1999 ZALESKA ET AL: "Apomorphine in treatment of Parkinson's disease with fluctuations" retrieved from MEDLIN Database accession no. nlm10791032 XP002204675 & ZALESKA ET AL: "Apomorphine in treatment of Parkinson's disease with fluctuations" NEUROLOGIA I NEUROCHIRURGIA POLSKA, vol. 33, no. 6, 1999, pages 1297-1303,
- GLASE ET AL: "SUBSTITUTED ((4-PHENYLPIPEUAZINYL)-METHYL)BENZAMIDES: SELECTIVE DOPAMINE D4 AGONISTS" J MED CHEM, vol. 40, 1997, pages 1771-1772, XP002204674 cited in the application

## Description

### TECHNICAL FIELD

The present invention relates to the use of selective dopamine D₄ receptor agonists and to compositions containing selective dopamine D₄ receptor agonists for the treatment of sexual dysfunction.

### BACKGROUND OF THE INVENTION

Penile flaccidity and erection are determined by the tone of the corpus cavernosum smooth muscle of the penis. The muscle tone is controlled by complex biochemical events coordinated at the level of the peripheral and central nervous system. Sympathetic, parasympathetic and somatic nerves control cavemosal tone via neuroanatomical connections that are an integral part of the innervation of the lower urinary tract.

Penile erection is the end result of cavemosal smooth muscle relaxation which can be initiated by central nervous system (CNS) pathways. These pathways activate peripheral nerves innervating the penis resulting in the release of nitric oxide (NO). Diffusion of NO mediates the relaxation of the cavemosal smooth muscle leading to penile erection.

Preclinical evidence indicates that dopamine (DA) plays a role in penile erection in mammals. Sexual stimulation can be initiated by sensory (erotic) information reaching the cerebral cortex in mammals. The cerebral cortex has extensive neuronal connections with limbic structures like the amygdala, as well as midbrain structures like the periaqueductal gray (PAG) and the hypothalamus. Two important nuclei in the hypothalamus are the medial preoptic area (MPOA) and the paraventricular nucleus (PVN). The MPOA and PVN nuclei play a critical role in sexual behavior as bilateral lesions of these areas completely eliminate male sexual behavior. The incerto-hypothalamic dopaminergic pathway that innervates the PVN and the MPOA nuclei has been associated with the pro-erectile effect of DA agents. Systemic administration of DA receptor agonists like apomorphine (5,6,6a,7-tetrahydro-6-methyl-4H-dibenzo[de,g]quinoline-10,11-diol), quinpirole and (-) 3-(3-hydroxyphenyl)-N-propylpiperidine (3-PPP) facilitate penile erection in rats, an effect blocked by haloperidol, a central DA antagonist. As the erectogenic effect can not be blocked by domperidone, a peripheral DA antagonist, it is believed that the pro-erectile effect of DA agonists is centrally mediated (Andersson K and Wagner G, Physiology of penile erection, Physiol Rev (1995) 75:191-236; deGroat W and Booth A, Neural Control of Penile Erection, in: Nervous control of urogenital system, Vol. 3, (ed. Maggi, C) (1993) p. 467-524, Hardwood Academic Publishers, Chur, Switzerland; and Moreland RB, Nakane M, Hsieh G and Brioni JD, Prospectives for Pharmacotherapy of Male Erectile Dysfunction, Curr Opinion CPNS Invest Drugs (2000) 2:283-302).

Clinical data also indicates that DA systems in the CNS play a role on the regulation of male sexual behavior as indicated by the sexual stimulatory effect of L-dopa in Parkinson's patients and by the pro-erectile effect of apomorphine in humans (Morales A, Geaton J, Johnston B and Adams M, Oral and Topical Treatment of Erectile Dysfunction: present and future, in: Urologic Clinics of North America, (1995) Vol. 22, p. 879-886; Padma-Nathan H, Auerbach S, Lewis R, Lewand M and Perdok R , Efficacy and safety of apomorphine SL vs. placebo for male erectile dysfunction (MED), Urology (1999) 161:214 (abstract 821); and Dula E, Keating W, Siami P, Edmonds A, O'Neil J, Efficacy and safety of fixed-dose and dose-optimization regimens of sublingual apomorphine versus placebo in men with erectile dysfunction, Urology (2000) 56:130-135).

DA receptors belong to a superfamily of protein receptors that signal across the cell membrane by coupling to intracellular GTP-binding proteins. Several G proteins have been identified (including Gs, Gq and Gi) that lead to specific intracellular events (Milligan G and Rees S, Chimaeric G proteins: their potential use in drug discovery, Trends Pharmacol Sci (1999) 20:118-124).

There are five known DA receptors which are classified into two groups, D₁-like and D₂-like. The D₁-like receptors include D₁ and D₅. The D₂-like receptors include D₂, D₃ and D₄ (Missale C, Nash S, Robinson S, Jaber M and Caron M, Dopamine receptors: from structure to function, Physiol Rev (1998) 78:189-225). The D₁-like family receptor subtypes are Gₛ-coupled and can activate adenylate cyclase. The D₂-like family receptor subtypes are Gᵢ-coupled and they increase intracellular calcium level and inhibit adenylate cyclase.

The D₁-like family members are Gₛ-coupled receptors that can activate adenylate cyclase. The D₁ receptor is the most abundant and widespread DA receptor in the CNS both by mRNA expression and by immunohistochemical studies (Vallone D, Picetti R and Borreli E, Structure and function of dopamine receptors, Neurosci Biobehav Rev (2000) 24:125-132). It is found in the striatum, nucleus accumbens and olfactory tubercle as well as the limbic system, hypothalamus and thalamus. The D₁ receptor expression has been reported in the heart and kidney, and despite that the function of these peripheral D₁ receptors remains to be clarified, its role on the control of hemodynamic variables has been confirmed. The D₅ receptor, while having a higher affinity for DA than the D₁ receptor, is sparsely distributed in the CNS with no evidence of expression outside the CNS.

The D₂-like family members are Gᵢ coupled receptors that inhibit adenylate cyclase and increase intracellular calcium levels. The D₂ receptor is the most abundant of the D₂-like receptors and is located in brain areas such as the striatum and substantia nigra, and in peripheral areas such as the heart, pituitary gland and kidney. The D₃ receptor is found abundantly in the islands of Calleja with distinct cluster populations in the ventral striatum/nucleus accumbens regions, olfactory tubercle, dendate gyrus and striatal cortex (Suzuki M, Hurd Y, SokoloffP, Schwartz J and Sedwall G, D₃ dopamine receptor mRNA is widely express in human brain, Brain Res (1998) 779:58-74).

Expression of the D₄ receptor has been documented by in situ RNA hybridization and immunohistochemical studies. Recently, studies revealed that D₄ expression is highest in the entorhinal cortex, lateral septal nucleus, hippocampus and the medial preoptic area of the hypothalamus (Primus R, Thurkauf A, Xu J, Yevich E, Mcinerney S, Shaw K, Tallman J and Gallagher D, Localization and characterization of dopamine D₄ binding sites in rat and human brain by use of the novel D₄ receptor-selective ligand [³H]NGD 94-1, J Pharmacol Exp Ther (1997) 282:1020-1027). Localization of D₄ is distinct from the distribution of D₂ in the brain, as D₂ receptors are most abundant in striatal areas. The expression of D₄ receptors in the MPOA of the hypothalamus is of importance to the facilitation of penile erection in view of the role of the hypothalamus as an area of integration between the cortex and the spinal pathways. The participation of D₄ receptors in other CNS regions, thalamic, subthalamic and spinal can not be excluded.

WO 96/04250 has disclosed benzimidazole derivatives having a preference for the D₄ dopamine receptor which are useful for treating a variety of central nervous system disorders by increasing or decreasing dopamine mediated neurotransmission in a mammal.

Zaleska, B., Abstract ofNeurologia i Neurochirurgia Polska, 1999, 33(6) have disclosed that apomorphine is used for treating Parkinson's disease and that it is a non-specific dopamine agonist which has a strong action on D₂, D₃ and D₄ receptors and a weaker action on D₁ and D₅ receptors. Domperidone has been concurrently administered for counteracting the emetic action of apomorphine.

WO 00/35457 has taught the use of apomorphine for the treatment of erectile dysfunction in males and U.S. Patent 5,945,117 has taught the use of apomorphine for the treatment of sexual dysfunction in females. Both of these documents indicate that apomorphine is a known emetic.

Two compounds, N-{[4-(2-cyanophenyl)-1-piperazinyl]methyl} -3-methylbenzamide and 5-fluoro-2-{[4-(2-pyridinyl)-1-piperazinyl]methyl}-1H-indole, have recently been described as selective dopamine D₄ receptor agonists (Glase SA, Akunne H, Georgic L, Heffner T, MacKenzie R, Manley P, Pugsley T and Wise L, J Med Chem (1997) 40:1771-1772; and Zorn SH, Jackson E, Johnson C, Lewis J, Fliri A, Soc Neurosci Abstr 23:685 (1997)). However, no specific therapeutic utility has been identified for compounds that are D₄ selective agonists.

The present invention identifies a therapeutic use for compounds that are D₄ agonists that are useful in the treatment of sexual dysfunction in mammals. More specifically, compounds that are selective dopamine D₄ receptor agonists are useful in the treatment of sexual dysfunction including, but not limited to, male erectile dysfunction (MED).
Dopamine receptors are know to mediate several other physiological responses such as emesis and hemodynamic effects. Accordingly, selective D₄ agonists offer an advantage over non-selective agents in that selective D₄ agonists reduce the incidence of emesis and/or hemodynamic effects in mammals.

### SUMMARY OF THE INVENTION

In its principle embodiment, the invention relates to the use of a dopamine D₄ receptor agonist or a pharmaceutically acceptable salt thereof for manufacturing a medicament for treating sexual dysfunction in a mammal by administration to a mammal in need of such treatment of a therapeutically effective amount, wherein said D₄ receptor agonist is at least 100 fold more selective for the D₄ receptor than for the D₂ receptor.

Another embodiment of the present invention relates to the use of a dopamine D₄ receptor agonist or a pharmaceutically acceptable salt thereof for manufacturing a medicament for treating male sexual dysfunction in a mammal, including, but not limited to, erectile dysfunction and premature ejaculation, by administration to a mammal in need of such treatment of a therapeutically effective amount, wherein said D₄ receptor agonist is at least 100 fold more selective for the D₄ receptor than for the D₂ receptor.

Another embodiment of the present invention relates to the use of a dopamine D₄ receptor agonist or a pharmaceutically acceptable salt thereof for manufacturing a medicament for treating female sexual dysfunction in a mammal, including, but not limited to, female anorgasmia, clitoral erectile insufficiency, vaginal engorgement, dyspareunia, and vaginismus, by administration to a mammal in need of such treatment of a therapeutically effective amount, wherein said D₄ receptor agonist is at least 100 fold more selective for the D₄ receptor than for the D₂ receptor.

### DETAILED DESCRIPTION OF THE INVENTION

In its principle embodiment, the invention relates to the use of a dopamine D₄ receptor agonist or a pharmaceutically acceptable salt thereof for manufacturing a medicament for treating sexual dysfunction in a mammal by administration to a mammal in need of such treatment of a therapeutically effective amount, wherein said D₄ receptor agonist is at least 100 fold more selective for the D₄ receptor than for the D₂ receptor. The present invention relates to the use of a dopamine D₄ receptor agonist or a pharmaceutically acceptable salt thereof for manufacturing a medicament for treating male sexual dysfunction in a mammal, including, but not limited to, erectile dysfunction and premature ejaculation, by administration to a mammal in need of such treatment of a therapeutically effective amount, wherein said D₄ receptor agonist is at least 100 fold more selective for the D₄ receptor than for the D₂ receptor. The present invention also relates to the use of a dopamine D₄ receptor agonist or a pharmaceutically acceptable salt thereof for manufacturing a medicament for treating female sexual dysfunction in a mammal, including, but not limited to, female anorgasmia, clitoral erectile insufficiency, vaginal engorgement, dyspareunia, and vaginismus, by administration to a mammal in need of such treatment of a therapeutically effective amount, wherein said D₄ receptor agonist is at least 100 fold more selective for the D₄ receptor than for the D₂ receptor.

A preferred embodiment of the present invention relates to the use of a dopamine D₄ receptor agonist or a pharmaceutically acceptable salt thereof for manufacturing a medicament for treating male erectile dysfunction in a mammal by administration to a mammal in need of such treatment of a therapeutically effective amount, wherein said D₄ receptor agonist is at least 100 fold more selective for the D₄ receptor than for the D₂ receptor.

Another embodiment of the present invention relates to the use of N-{[4-(2-cyanophenyl)-1-piperazinyl]methyl}-3-methylbenzamide or a pharmaceutically acceptable salt thereof for manufacturing a medicament for treating male sexual dysfunction in a mammal by administration to a mammal in need of such treatment of a therapeutically effective amount.

Another embodiment of the present invention relates to the use of N-{[4-(2-cyanophenyl)-1-piperazinyl]methyl}-3-methylbenzamide or a pharmaceutically acceptable salt thereof for manufacturing a medicament for treating female sexual dysfunction in a mammal by administration to a mammal in need of such treatment of a therapeutically effective amount.

Another embodiment of the present invention relates to the use of N-{[4-(2-cyanophenyl)-1-piperazinyl]methyl}-3-methylbenzamide or a pharmaceutically acceptable salt thereof for manufacturing a medicament for treating male erectile dysfunction in a mammal by administration to a mammal in need of such treatment of a therapeutically effective amount.

Another embodiment of the present invention relates to the use of 5-fluoro-2-{[4-(2-pyridinyl)-1-piperazinyl]methyl}-1H-indole or a pharmaceutically acceptable salt thereof for manufacturing a medicament for treating male sexual dysfunction in a mammal by administration to a mammal in need of such treatment of a therapeutically effective amount.

Another embodiment of the present invention relates to the use of 5-fluoro-2-{[4-(2-pyridinyl)-1-piperazinyl]methyl}-1H-indole or a pharmaceutically acceptable salt thereof for manufacturing a medicament for treating female sexual dysfunction in a mammal by administration to a mammal in need of such treatment of a therapeutically effective amount.

Another embodiment of the present invention relates to the use of 5-fluoro-2-{[4-(2-pyridinyl)-1-piperazinyl]methyl}-1H-indole or a pharmaceutically acceptable salt thereof for manufacturing a medicament for treating male erectile dysfunction in a mammal by administration to a mammal in need of such treatment of a therapeutically effective amount.

In another embodiment of the present invention, the selective dopamine D₄ receptor agonist is a compound which is a) at least 200 fold selective in terms of Kᵢ value for the dopamine D₄ receptor compared with the Kᵢ value for the dopamine D₂ receptor; and b) acts as an agonist in any one of the functional pharmacological models of the dopamine D₄ receptor as described herein.

In another embodiment of the present invention, the selective dopamine D₄ receptor agonist is a compound which is a) at least 300 fold selective in terms of Kᵢ value for the dopamine D₄ receptor compared with the Kᵢ value for the dopamine D₂ receptor; and b) acts as an agonist in any one of the functional pharmacological models of the dopamine D₄ receptor as described herein.

In another embodiment of the present invention, the selective dopamine D₄ receptor agonist is a compound which is a) at least 500 fold selective in terms of Kᵢ value for the dopamine D₄ receptor compared with the Kᵢ value for the dopamine D₂ receptor; and b) acts as an agonist in any one of the functional pharmacological models of the dopamine D₄ receptor as described herein.

In another embodiment of the present invention, the selective dopamine D₄ receptor agonist is a compound which is a) at least 1000 fold selective in terms of Kᵢ value for the dopamine D₄ receptor compared with the Kᵢ value for the dopamine D₂ receptor; and b) acts as an agonist in any one of the functional pharmacological models of the dopamine D₄ receptor as described herein.

In the above embodiments according to the present invention, the selective dopamine D₄ receptor agonist or pharmaceutically acceptable salt thereof may be used in combination with a pharmaceutically acceptable carrier.

The term "selective", as used herein, refers to selectivity of a dopamine agonist for the D₄ receptor as compared to the D₂ receptor.

(-) Apomorphine, a known dopamine agonist (Altar, C, et al., Mol Pharmacol (1988) 33(6) 690-695; Moeller, HG et al., Psychopharmacology (Berlin) (1987) 91(1) 50-55; and Fray, PJ et al., Psychopharmacology (Berlin) (1980) 69(3) 253-259), was shown not to be selective for the dopamine D₄ receptor, Kᵢ = 4 nM, versus the dopamine D₂ receptor, Kᵢ = 0.7-24 nM (Vallone D et al., Neuroscience And Biobehavioral Reviews, (2000) 24, 125-132).

The lack of selectivity of dopaminergic agonists like apomorphine helps explain the side effects such as emesis and syncope which are associated with these agents. The use of selective D₄ agonists represents a significant advantage for the treatment of CNS mediated disorders as selective D₄ agonists facilitate penile erections without inducing side effects such as emesis and syncope. The term "significant emesis" as used herein refers to an emetic response of 20% or more that is observed in a clinical population. Preferably, the emetic response in a clinical population is less than 10%. More preferably, the emetic response in a clinical population is less than 5%.

### Dopamine D₂ and Dopamine D₄ Binding Assay

The dopamine D₂ and D₄ binding assays, used to determine binding affinities (Kᵢ) illustrated in Table 1, were conducted according to standard procedures known to those in the art and are summarized herein. The binding data presented in Table 1 were conducted by Cerep, 86600 Celle L'Evescault, France.

Briefly, receptor binding assays at the dopamine D₂ and D₄ receptors were carried out in membranes from cell lines expressing the recombinant human subtypes. For the D₂ receptor assay, membranes of transfected A9L cells were incubated with [³H]-spiperone (0.3 nM) for 60 minutes, using (+)-butaclamol (10 µM) to define nonspecific binding (Grandy, et al., Proc Natl Acad Sci USA (1989) 86:9762-9766). For the D₄ receptor assay, membranes of transfected CHO cells were incubated with [³H]-spiperone (0.5 nM) for 60 minutes, using (+)-butaclamol (10 µM) to define nonspecific binding (Van Tol, et al., Nature (1992) 358: 149-152). Specific binding was defined as the difference between total binding and nonspecific binding. Kᵢ values were determined using the Cheng-Prusoff equation and are illustrated in Table 1.

### Binding Data for Dopamine D₂ and D₄ Receptors

The data in Table 1 demonstrates the > 100 fold selectivity of N-{[4-(2-Cyanophenyl)-1-piperazinyl]methyl}-3-methylbenzamide and 5-Fluoro-2-{[4-(2-pyridinyl)-1-piperazinyl]methyl}-1H-indole for dopamine D₄ receptors when compared to dopamine D₂ receptors.

**Table 1**

| Compound | Kᵢ (nM) | |
|---|---|---|
| | D₂ | D₄ |
| Apomorphine | 32 | 1.5 |
| N-{[4-(2-Cyanophenyl)-1-piperazinyl]methyl}-3-methylbenzamide | >1000 | 10 |
| 5-Fluoro-2-{[4-(2-pyridinyl)-1-piperazinyl]methyl}-1H-indole | >1000 | 2.6 |

N-{[4-(2-Cyanophenyl)-1-piperazinyl]methyl}-3-methylbenzamide has been shown to be >400 fold selective for the dopamine D₄ receptor, Kᵢ = 8.7 nM, compared to the dopamine D₂ receptor, Kᵢ = 3740 nM (Glase, SA et al., J Med Chem (1997) 40, 1771-1772; and Chio, C et al., Mol Pharmacol (1994) 45, 51-60).

5-Fluoro-2-{[4-(2-pyridinyl)-1-piperazinyl]methyl}-1H-indole has been shown to be >100 fold selective for the dopamine D₄ receptor, Kᵢ = 6.0 nM, compared to the dopamine D₂ receptor (Zorn SH, et al., Soc Neurosci Abstr 23:685 (1997)).

The term "agonist", as used herein, refers to a chemical entity that interacts with a receptor and elicits an observable biochemical response. The response is measured relative to a known agonist standard. For example, a dopamine D₄ receptor agonist refers to a chemical entity that interacts with the dopamine D₄ receptor and elicits an observable biochemical response. The response is measured relative to a full agonist such as dopamine or quinpirole.

The determination of dopamine D₄ receptor agonism can be established by any one of the three assays described below. A compound that shows a minimum of 25% agonism at the dopamine D4 receptor in any one of the three assays described below, as compared to dopamine or quinpirole as the 100% agonist standard, is considered an agonist within the scope of the present invention. The percent agonism a value that is based on the mean of at least three independent observations. Preferably, D₄ agonists contemplated within the scope of the present invention will show 40% agonism or greater as compared to a dopamine or quinpirole standard.

### Functional Pharmacological Models of Dopamine D4 Receptor Agonism

### 1. [³H]thymidine assay

Agonist activation of dopamine D₄ receptors in CHO pro-5 cells transfected with human dopamine D₄ receptor stimulates mitogenesis. The response is determined by measuring the cellular uptake of [³H]thymidine and comparing the response to a full agonist such as quinpirole (defined as 100%). N-{[4-(2-Cyanophenyl)-1-piperazinyl]methyl}-3-methylbenzamide was shown to elicit a response in the [³H]thymidine uptake assay, eliciting an 80% response in comparison to quinpirole with an EC₅₀ of 17 nM (Glase, SA et al., J Med Chem (1997) 40, 1771-1772; and Chio, C et al., Mol Pharmacol (1994) 45, 51-60).

CHO pro-5 cells transfected with the human D₄ receptor are plated on 96-well plates in MEM-Alpha with 10% fetal calf serum containing penicillin (100 U/mL) and streptomycin (100 g/mL). Forty-eight hours later, cells are serum deprived by washing and maintaining in serum-free media. Twenty-four hours later, vehicle, standards, or test compounds are added. Eighteen hours later, [³H]thymidine (5 µCi/well) is added for 2 hours, then trypsin (100 µL of 0.25%) is added for 1 hour, and the assay is terminated by filtration using a Brandel or other 96-well harvester. The filters are counted for radioactivity using the LKB-plate counting system or other plate counting system. Ten-point dose-response curves are determined for each test compound, and the drug concentration necessary for 50% stimulation (EC₅₀) is calculated from the resulting curve. Intrinsic agonist activity is assessed by comparing the maximal effect of each test compound relative to the effect obtained with a maximally effective concentration of quinpirole in each experiment.

### 2. cAMP assay

The cAMP assay is a functional assay for determining dopamine D₄ functional activity that involves measuring the inhibition of forskolin-stimulated cAMP accumulation in CHO cells expressing the human D₄ receptor. Agonists inhibit accumulation of forskolin-stimulated cAMP. 5-Fluoro-2-{[4-(2-pyridinyl)-1-piperazinyl]methyl}-1H-indole was shown to inhibit accumulation of forskolin-stimulated cAMP, EC₅₀= 5.8 nM (Zorn SH, et al., Soc Neurosci Abstr 23:685 (1997)).

The cAMP assay that is used for measuring the inhibition of forskolin-stimulated cAMP accumulation is described in Gazi et al., Arch Pharmacol. 361 (2000) 555-564. Cells are grown to confluence in 24-well plates and are washed with 1 ml of Hepes-buffered salt solution. The cells are then labeled with 6 µCi/ml of [2-³H]adenine (23 Ci/mmol; Anawa Trading, Wangen, Switzerland) at 37 °C for 2 hours in 0.5 ml of the same buffer. They are then washed twice with 1 ml of the buffer solution that is supplemented with 1 mM isobutylmethylxanthine. The cells are incubated in 1 ml of the same solution at 37 °C, in the presence and absence of forskolin (10 µM) and test compounds. After 15 minutes, the medium is removed and is replaced by 1 ml 5% trichloroacetic acid solution containing cAMP and ATP (both 0.1 mM). After 30 minutes at 4 °C, the trichloroacetic acid extracts are directly subjected to sequential chromatography on Dowex AG 50W-X4 and alumina columns. cAMP formation is calculated as the ratio [³H]cAMP/([³H]cAMP+[³H]ATP).

### 3. Fluorescence Image Plate Reader (FLIPR) assay

The FLIPR assay is a functional assay that can be conducted in HEK293 cells expressing the human D₄ receptor, co-transfected with the G_{qo5} protein chimera. Expression of the G-protein chimera allows for the mobilization of intracellular calcium after activation of the D₄ receptor. Cells are prepared by plating the stably transfected D₄-HEK293 cells into each well of 96 well plates. Cells are cultured until they reach confluence (approximately 48 hours). Media is removed from the plate and media containing the fluorescent calcium detecting dye Fluo-4 is added. Cells are incubated at room temperature for one hour. Cells are then washed three times with phosphate buffered saline and 150 µl PBS is added. Compounds to be tested are diluted in a second 96-well plate. The plate containing the cells is placed in fluorescent plate reader (FLIPR) and the reaction is begun by adding 50 µl of compound solution to all the wells simultaneously. Fluorescent signal is measured for 60 seconds sampling at 1 second intervals. The signal derived from 10 µM dopamine is used as 100% and a compound dose-response curve is normalized to determine the percent efficacy relative to dopamine. Dose response curves are analyzed to determine EC₅₀ (nM). Data for the N-{[4-(2-cyanophenyl)-1-piperazinyl]methyl}-3-methylbenzamide and 5-fluoro-2-{[4-(2-pyridinyl)-1-piperazinyl]methyl}-1H-indole are shown in Table A. The data in Table A demonstrates that N-{[4-(2-cyanophenyl)-1-piperazinyl]methyl}-3-methylbenzamide and 5-fluoro-2-{[4-(2-pyridinyl)-1-piperazinyl]methyl}-1H-indole are agonists.

**Table A**

| | n | EC₅₀ (nM) | Maximum Response |
|---|---|---|---|
| N-{[4-(2-cyanophenyl)-1-piperazinyl]methyl}-3-methylbenzamide | 12 | 5.6 | 68% |
| 5-fluoro-2-{[4-(2-pyridinyl)-1-piperazinyl]methyl}-1H-indole | 4 | 32 | 46 % |

### Rat Penile Erection Model

Wistar rats were used as a primary animal model to study penile erection in vivo. All experiments were carried out between 9:00 AM and 3:00 PM in a diffusely illuminated testing room with a red light. The rats were weighed and allowed to adapt to the testing room for 60 minutes before the beginning of experiments. Rats were placed in individual transparent cages (20x30x30 cm) after subcutaneous drug injection. The number of penile erections was recorded by direct observation for a period of 60 minutes after drug dosing. The number of animals exhibiting 1 or more erections was recorded and expressed as incidence (%) in Tables 2-4.

### Apomorphine Induced Penile Erections in Rats

(L)-Ascorbic acid in saline (1 mg/mL) was used as vehicle. Thirty two animals were used per dose. The data in Table 2 demonstrates that apomorphine induced a significant facilitation of penile erections in rats for doses 0.01 µmol/kg to 0.3 µmol/kg. The probability or significance level is represented by p versus vehicle. "p" is the probability or significance level in a statistical test.

**Table 2**

| Dose µmol/kg | Incidence (%) | p |
|---|---|---|
| vehicle | 22 | |
| 0.003 | 28 | |
| 0.01 | 56 | <0.01 |
| 0.03 | 69 | <0.001 |
| 0.1 | 92 | <0.001 |
| 0.3 | 66 | <0.001 |
| 1.0 | 25 | |

### N- {[4-(2-Cyanophenyl)-1-piperazinyl]methyl} -3-methylbenzamide

### Induced Penile Erections in Rats

(L)-Ascorbic acid in saline (1mg/mL) was used as vehicle. Fourteen animals were used per dose. Apomorphine was used as a positive control at a dose of 0.1 µmol/kg which resulted in an 86 % incidence of rat penile erections. The data in Table 3 demonstrates that N-{[4-(2-Cyanophenyl)-1-piperazinyl]methyl}-3-methylbenzamide induced a significant facilitation of penile erections in rats for doses 0.1 µmol/kg to 1.0 µmol/kg. The probability or significance level is represented by p as compared to vehicle.

**Table 3**

| Dose µmol/kg | Incidence (%) | p |
|---|---|---|
| vehicle | 20 | |
| apomorphine (0.1) | 86 | <0.001 |
| 0.03 | 42 | |
| 0.1 | 71 | <0.01 |
| 0.3 | 79 | <0.01 |
| 1.0 | 68 | <0.05 |

### 5-Fluoro-2-{[4-(2-pyridinyl)-1-piperazinyl]methyl}-1H-indole

### Induced Penile Erections in Rats

(L)-Ascorbic acid in saline (1 mg/mL) was used as vehicle. Ten animals were used per dose. Apomorphine was used as a positive control at a dose of 0.1 µmol/kg which resulted in an 90% incidence of rat penile erections. The data in Table 4 demonstrates that 5-fluoro-2-{[4-(2-pyridinyl)-1-piperazinyl]methyl}-1H-indole induced a significant facilitation of penile erections in rats for doses 1.0 µmol/kg and 3.0 µmol/kg. The probability or significance level is represented by p as compared to vehicle.

**Table 4**

| Dose µmol/kg | Incidence (%) | p |
|---|---|---|
| vehicle | 40 | |
| apomorphine (0.1) | 90 | <0.01 |
| 0.3 | 60 | |
| 1.0 | 90 | <0.01 |
| 3.0 | 90 | <0.01 |

### Emesis Model in Ferrets

Male Fitch ferrets (body weights 1.0-1.5 kg) were fasted overnight before experimentation. Animals were placed individually in observation cages following subcutaneous administration of drug and observed for drug-induced nausea and emesis for a period of 90 minutes following drug injection. Nausea was characterized by behaviors such as licking, gagging, backing, head burying, and intense abdominal grooming. Emesis was usually preceded by these behaviors and was characterized by rhythmic abdominal contractions which were associated with vomiting or retching movement. The number of ferrets induced to emesis was recorded and expressed as incidence (%) in Tables 5-7.

### Apomorphine Induced Emesis in Rats

Saline was used as vehicle. Six to twelve animals were used per dose. The data in Table 5 demonstrates that apomorphine induced emesis at all doses. The probability or significance level is represented by p as compared to vehicle.

**Table 5**

| Dose µmol/kg | Incidence (%) | p |
|---|---|---|
| vehicle | 0 | |
| 0.1 | 32 | <0.05 |
| 0.3 | 82 | <0.001 |
| 1.0 | 48 | <0.01 |
| 3.0 | 32 | <0.05 |
| 10.0 | 32 | <0.05 |

### N-{[4-(2-Cyanophenyl)-1-piperazinyl]methyl}-3-methylbenzamide

### Induced Emesis in Rats

3% Dimethylsulfoxide in saline was used as vehicle. Six to twelve animals were used per dose. Apomorphine was used as a positive control in Table 6 at a dose of 0.3 µmol/kg which resulted in an 83 % incidence of ferrets induced to emesis. N-{[4-(2-Cyanophenyl)-1-piperazinyl]methyl}-3-methylbenzamide did not induce emesis at any dose. The probability or significance level is represented by p as compared to vehicle.

**Table 6**

| Dose µmol/kg | Incidence (%) | p |
|---|---|---|
| vehicle | 0 | |
| apomorphine (0.3) | 83 | <0.001 |
| 0.1 | 0 | |
| 0.3 | 0 | |
| 1.0 | 0 | |
| 3.0 | 0 | |

### 5-Fluoro-2-{[4-(2-pyridinyl)-1-piperazinyl]methyl}-1H-indole

### Induced Emesis in Rats

1% Dimethylsulfoxide in saline was used as vehicle. Six to twelve animals were used per dose. Apomorphine was used as a positive control in Table 7 at a dose of 0.3 µmol/kg which resulted in an 83 % incidence of ferrets induced to emesis. 5-Fluoro-2-{[4-(2-pyridinyl)-1-piperazinyl]methyl}-1H-indole did not induce emesis at any dose. The probability or significance level is represented by p as compared to vehicle.

**Table 7**

| Dose µmol/kg | Incidence (%) | p |
|---|---|---|
| vehicle | 0 | |
| apomorphine (0.3) | 83 | <0.001 |
| 0.1 | 0 | |
| 0.3 | 0 | |
| 1.0 | 0 | |
| 3.0 | 0 | |

The data in Tables 2-7 indicate that nonselective and selective dopamine D₄ receptor agonists have a pro-erectile effect in rats. However, selective dopamine D₄ receptor agonists have a significantly reduced emetic liability. The pro-erectile effect and reduced emetic liability associated with selective dopamine D₄ receptor agonists, therefore, suggests that selective dopamine D₄ receptor agonists are useful for the treatment of sexual dysfunction including, but not limited to, male erectile dysfunction.

The term "pharmaceutically acceptable carrier," as used herein, means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The present invention uses pharmaceutical compositions which comprise selective dopamine D₄ receptor agonists formulated together with one or more non-toxic pharmaceutically acceptable carriers. The pharmaceutical compositions can be formulated for oral administration in solid or liquid form, for parenteral injection or for rectal administration.

Further included within the scope of the present invention is the use of pharmaceutical compositions comprising one or more selective dopamine D₄ receptor agonists prepared and formulated in combination with one or more non-toxic pharmaceutically acceptable compositions. For example, pharmaceutical compositions comprising one or more selective dopamine D₄ receptor agonists can be formulated in combination with a phosphodiesterase 5 inhibitor or an adrenoceptor antagonist. The pharmaceutical compositions can be formulated for sublingual dosage, oral administration in solid or liquid form, for parenteral injection, or for rectal administration.

The pharmaceutical compositions can be administered to humans and other mammals orally, sublingually, rectally, parenterally , intracisternally, intraurethrally, intravaginally, intraperitoneally, topically (as by powders, ointments or drops), bucally or as an oral or nasal spray. The term "parenterally," as used herein, refers to modes of administration which include intravenous, intramuscular, intraperitoneal, subcutaneous, intraarticular injection and infusion.

Sublingual compositions can be an effective dosage form in treating sexual dysfunction and sublingual compositions are well documented in the literature. Traditional sublingual tablets are usually designed as water soluble, although less soluble tablets are possible. Time release sublingual medications are disclosed in U.S. Pat. No. 3,428,728.

Pharmaceutical compositions for parenteral injection comprise pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity may be maintained, for example, by the use of a coating such as lecithin, by the maintenance of the required particle size in the case of dispersions, and by the use of surfactants.

These compositions may also contain adjuvants such as preservative agents, wetting agents, emulsifying agents, and dispersing agents. Prevention of the action of microorganisms may be ensured by various antibacterial and antifungal agents, for example, parabens, chlorobutanol, phenol, sorbic acid, and the like. It may also be desirable to include isotonic agents, for example, sugars, sodium chloride and the like. Prolonged absorption of the injectable pharmaceutical form may be brought about by the use of agents delaying absorption, for example, aluminum monostearate and gelatin.

In some cases, in order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Suspensions, in addition to the active compounds, may contain suspending agents, as, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

If desired, and for more effective distribution, selective dopamine D₄ receptor agonists can be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

Selective dopamine D₄ receptor agonists can also be in micro-encapsulated form, if appropriate, with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound can be admixed with at least one inert diluent such as sucrose, lactose, or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of such composition that they release the active ingredient(s) only, or preferentially, in a certain part of the intestinal tract in a delayed manner. Examples of embedding compositions which can be used include polymeric substances and waxes.

Injectable depot forms are made by forming microencapsulated matrices of the selective dopamine D₄ receptor agonist in biodegradable polymers such as polylactide-polyglycolide. Depending upon the ratio of selective dopamine D₄ receptor agonist to polymer and the nature of the particular polymer employed, the rate of selective dopamine D₄ receptor agonist release can be controlled. Examples of other biodegradable polymers include poly(orthoesters) and poly(anhydrides) Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic, parenterally acceptable diluent or solvent such as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Solid dosage forms for oral administration include capsules, tablets, pills, powders, and granules. In such solid dosage forms, the selective dopamine D₄ receptor agonist is mixed with at least one inert, pharmaceutically acceptable excipient or carrier such as sodium citrate or dicalcium phosphate and/or a) fillers or extenders such as starches, lactose, sucrose, glucose, mannitol, and silicic acid; b) binders such as carboxymethylcellulose, alginates, gelatin, polyvinylpyrrolidinone, sucrose, and acacia; c) humectants such as glycerol; d) disintegrating agents such as agar-agar, calcium carbonate, potato or tapioca starch, alginic acid, certain silicates, and sodium carbonate; e) solution retarding agents such as paraffin; f) absorption accelerators such as quaternary ammonium compounds; g) wetting agents such as cetyl alcohol and glycerol monostearate;) absorbents such as kaolin and bentonite clay; and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract in a delayed manner. Examples of embedding compositions which can be used include polymeric substances and waxes.

Liquid dosage forms for oral administration include pharmaceutically acceptable emulsions, microemulsions, solutions, suspensions, syrups and elixirs. In addition to the selective dopamine D₄ receptor agonist, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to a selective dopamine D₄ receptor agonist, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to selective dopamine D₄ receptor agonist, excipients such as lactose, talc, silicic acid, aluminum hydroxide, calcium silicates and polyamide powder, or mixtures of these substances. Sprays can additionally contain customary propellants such as chlorofluorohydrocarbons.

Selective dopamine D₄ receptor agonists can be used in the form of pharmaceutically acceptable salts derived from inorganic or organic acids. By "pharmaceutically acceptable salt" is meant those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge et al. describe pharmaceutically acceptable salts in detail in J. Pharmaceutical Sciences, 1977, 66: 1 et seq. The salts can be prepared in situ during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate, hydrochloride, hydrobromide, hydroiodide, 2-hydroxyethansulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate.

The term "pharmaceutically acceptable prodrug" or "prodrug,"as used herein, represents those prodrugs of selective dopamine D₄ receptor agonists which are, within the scope of sound medical judgement, suitable for use in contact with the tissues of humans and lower animals without undue toxicity, irritation, allergic response, and the like, commensurate with a reasonable benefit/risk ratio, and effective for their intended use. Prodrugs of selective dopamine D₄ receptor agonists may be transformed in vivo to selective dopamine D₄ receptor agonists, for example, by hydrolysis in blood. A thorough discussion is provided in T. Higuchi and V. Stella, Pro-drugs as Novel Delivery Systems, V. 14 of the A.C.S. Symposium Series, and in Edward B. Roche, ed., Bioreversible Carriers in Drug Design, American Pharmaceutical Association and Pergamon Press (1987).

Dosage forms for topical administration of selective dopamine D₄ receptor agonists include powders, sprays, ointments and inhalants. The active compound is mixed under sterile conditions with a pharmaceutically acceptable carrier and any needed preservatives, buffers or propellants which can be required. Opthalmic formulations, eye ointments, powders and solutions are also contemplated as being within the scope of this invention.

Actual dosage levels of active ingredients in the pharmaceutical compositions of this invention can be varied so as to obtain an amount of the selective dopamine D₄ receptor agonist(s) which is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the selective dopamine D₄ receptor agonist at levels lower than required for to achieve the desired therapeutic effect and to gradually increase the dosage until the desired effect is achieved.

The present invention contemplates selective dopamine D₄ receptor agonists either chemically synthesized or formed by in vivo biotransformation to selective dopamine D₄ receptor agonists.

When used in the above or other treatments, a therapeutically effective amount of a selective dopamine D₄ receptor agonist can be employed in pure form or, where such forms exist, in pharmaceutically acceptable salt or prodrug form. Alternatively, the selective dopamine D₄ receptor agonist can be administered as a pharmaceutical composition containing the selective dopamine D₄ receptor agonist of interest in combination with one or more pharmaceutically acceptable excipients. The phrase "therapeutically effective amount" of the compound of the invention means a sufficient amount of the selective dopamine D₄ receptor agonist to treat sexual dysfunction, at a reasonable benefit/risk ratio applicable to any medical treatment. It will be understood, however, that the total daily usage of the selective dopamine D₄ receptor agonist and compositions thereof will be decided by the attending physician within the scope of sound medical judgement. The specific therapeutically effective dose level for any particular patient will depend upon a variety of factors including the sexual dysfunction being treated and the severity of the sexual dysfunction; activity of the specific selective dopamine D₄ receptor agonist employed; the specific composition employed; the age, body weight, general health, sex and diet of the patient; the time of administration, route of administration, and rate of excretion of the specific selective dopamine D₄ receptor agonist employed; the duration of the treatment; drugs used in combination or coincidental with the specific selective dopamine D₄ receptor agonist employed; and like factors well known in the medical arts. For example, it is well within the skill of the art to start doses of selective dopamine D₄ receptor agonist at levels lower than required to achieve the desired therapeutic effect and to gradually increase the dosage until the desired effect is achieved.

The total daily dose of selective dopamine D₄ receptor agonist administered to a human or lower animal may range from about 0.001 to about 30 mg/kg/day. For purposes of oral administration, more preferable doses can be in the range of from about 0.01 to about 10 mg/kg/day. If desired, the effective daily dose can be divided into multiple doses for purposes of administration; consequently, single dose compositions may contain such amounts or submultiples thereof to make up the daily dose.

## Claims

1. Use of a dopamine D₄ receptor agonist or a pharmaceutically acceptable salt thereof for manufacturing a medicament for treating sexual dysfunction in a mammal by administration to a mammal in need of such treatment of a therapeutically effective amount, wherein said D₄ receptor agonist is at least 100 fold more selective for the D₄ receptor than for the D₂ receptor.

2. Use according to claim 1 of a dopamine D₄ receptor agonist or a pharmaceutically acceptable salt thereof for manufacturing a medicament for treating male sexual dysfunction in a mammal by administration to a mammal in need of such treatment of a therapeutically effective amount, wherein said D₄ receptor agonist is at least 100 fold more selective for the D₄ receptor than for the D₂ receptor.

3. Use according to claim 1 of a dopamine D₄ receptor agonist or a pharmaceutically acceptable salt thereof for manufacturing a medicament for treating female sexual dysfunction in a mammal by administration to a mammal in need of such treatment of a therapeutically effective amount, wherein said D₄ receptor agonist is at least 100 fold more selective for the D₄ receptor than for the D₂ receptor.

4. Use of a dopamine D₄ receptor agonist or a pharmaceutically acceptable salt thereof for manufacturing a medicament for treating male erectile dysfunction in a mammal by administration to a mammal in need of such treatment of a therapeutically effective amount, wherein said D₄ receptor agonist is at least 100 fold more selective for the D₄ receptor than for the D₂ receptor.

5. The use according to any one of Claims 1-4 wherein said selective dopamine D₄ receptor agonist is at least 200 fold more selective for the D₄ receptor than for the D₂ receptor.

6. The use according to any one of Claims 1-4 wherein said selective dopamine D₄ receptor agonist is at least 300 fold more selective for the D₄ receptor than for the D₂ receptor.

7. The use according to any one of Claims 1-4 wherein said selective dopamine D₄ receptor agonist is at least 500 fold more selective for the D₄ receptor than for the D₂ receptor.

8. The use according to any one of Claims 1-4 wherein said selective dopamine D₄ receptor agonist is at least 1000 fold more selective for the D₄ receptor than for the D₂ receptor.

9. The use according to any one of Claims 1-4 wherein said selective dopamine D₄ receptor agonist does not cause significant emesis.

10. The use according to any one of Claims 1-4 wherein said dopamine D₄ receptor agonist or a pharmaceutically acceptable salt thereof is in combination with a pharmaceutically acceptable carrier.

11. The use according to Claim 4 of one or more selective dopamine D₄ receptor agonists or a pharmaceutically acceptable salt thereof in combination with a phosphodiesterase 5 inhibitor or an adrenoreceptor antagonist.

12. The use according to any one of Claims 2-4 wherein said selective dopamine D₄ receptor agonist is N-{[4-(2-cyanophenyl)-1-piperazinyl]methyl}-3-methylbenzamide or a pharmaceutically acceptable salt thereof.

13. The use according to any one of Claims 2-4 wherein said selective dopamine D₄ receptor agonist is 5-fluoro-2-{[4-(2-pyridinyl)-1-piperazinyl]methyl}-1H-indole or a pharmaceutically acceptable salt thereof.

## Patentansprüche

1. Verwendung eines Dopamin D₄ Rezeptoragonisten oder eines pharmazeutisch verträglichen Salzes davon zur Herstellung eines Medikaments zur Behandlung von sexueller Dysfunktion in einem Säugetier durch Verabreichung einer therapeutisch wirksamen Menge an ein Säugetier, das eine solche Behandlung benötigt, worin der D₄ Rezeptoragonist mindestens 100 mal selektiver für den D₄ Rezeptor als für den D₂ Rezeptor ist.

2. Verwendung gemäß Anspruch 1 eines Dopamin D₄ Rezeptoragonisten oder eines pharmazeutisch verträglichen Salzes davon für die Herstellung eines Medikaments zur Behandlung männlicher sexueller Dysfunktion in einem Säugetier durch Verabreichung einer therapeutisch wirksamen Menge an ein Säugetier, das eine solche Behandlung benötigt, worin der D₄ Rezeptoragonist mindestens 100 mal selektiver für den D₄ Rezeptor als für den D₂ Rezeptor ist.

3. Verwendung gemäß Anspruch 1 eines Dopamin D₄ Rezeptoragonisten oder eines pharmazeutisch verträglichen Salzes davon für die Herstellung eines Medikaments zur Behandlung weiblicher sexueller Dysfunktion in einem Säugetier durch Verabreichung einer therapeutisch wirksamen Menge an ein Säugetier, das eine solche Behandlung benötigt, worin der D₄ Rezeptoragonist mindestens 100 mal selektiver für den D₄ Rezeptor als für den D₂ Rezeptor ist.

4. Verwendung eines Dopamin D₄ Rezeptoragonisten oder eines pharmazeutisch verträglichen Salzes davon für die Herstellung eines Medikaments zur Behandlung von männlicher erektiler Dysfunktion in einem Säugetier durch Verabreichung einer therapeutisch wirksamen Menge an ein Säugetier, das eine solche Behandlung benötigt, worin der D₄ Rezeptoragonist mindestens 100 mal selektiver für den D₄ Rezeptor als für den D₂ Rezeptor ist.

5. Die Verwendung gemäß irgendeinem der Ansprüche 1-4, worin der selektive Dopamin D₄ Rezeptoragonist mindestens 200 mal selektive für den D₄ Rezeptor als für den D₂ Rezeptor ist.

6. Die Verwendung gemäß irgendeinem der Ansprüche 1-4, worin der selektive Dopamin D₄ Rezeptoragonist mindestens 300 mal selektiver für den D₄ Rezeptor als für den D₂ Rezeptor ist.

7. Die Verwendung gemäß irgendeinem der Ansprüche 1-4, worin der selektive Dopamin D₄ Rezeptoragonist mindestens 500 mal selektiver für den D₄ Rezeptor als für den D₂ Rezeptor ist.

8. Die Verwendung gemäß irgendeinem der Ansprüche 1-4, worin der selektive Dopamin D₄ Rezeptoragonist mindestens 1000 mal selektiver für den D₄ Rezeptor als für den D₂ Rezeptor ist.

9. Die Verwendung gemäß irgendeinem der Ansprüche 1-4, worin der selektive Dopamin D₄ Rezeptoragonist kein signifikantes Erbrechen verursacht.

10. Die Verwendung gemäß irgendeinem der Ansprüche 1-4, worin der Dopamin D₄ Rezeptoragonist oder ein pharmazeutisch verträgliches Salz davon in Kombination mit einem pharmazeutisch verträglichen Träger vorliegt.

11. Die Verwendung gemäß Anspruch 4 eines oder mehrerer selektiver Dopamin D₄ Rezeptoragonisten oder eines pharmazeutisch verträglichen Salzes davon in Kombination mit einem Phosphodiesterase-5-Hemmer oder einem Adrenorezeptorantagonisten.

12. Die Verwendung gemäß irgendeinem der Ansprüch 2-4, worin der selektive Dopamin D₄ Rezeptoragonist N-{[4-(2-Cyanophenyl)-1-piperazinyl]methyl}-3-methylbenzamid oder ein pharmazeutisch verträgliches Salz davon ist.

13. Die Verwendung gemäß irgendeinem der Ansprüch 2-4, worin der selektive Dopamin D₄ Rezeptoragonist 5-Fluor-2-{ [4-(2-Pyridinyl)-1-piperazinyl]methyl}-1H-indol oder ein pharmazeutisch verträgliches Salz davon ist.

## Revendications

1. Utilisation d'un agoniste de récepteur D₄ de la dopamine ou d'un sel pharmaceutiquement acceptable de celui-ci pour la fabrication d'un médicament destiné à traiter un dysfonctionnement sexuel chez un mammifère par administration à un mammifère en besoin d'un tel traitement d'une quantité thérapeutiquement efficace, dans laquelle ledit agoniste de récepteur D₄ est au moins 100 fois plus sélectif pour le récepteur D₄ que pour le récepteur D₂.

2. Utilisation selon la revendication 1 d'un agoniste de récepteur D₄ de la dopamine ou d'un sel pharmaceutiquement acceptable de celui-ci pour la fabrication d'un médicament destiné à traiter un dysfonctionnement sexuel masculin chez un mammifère par administration à un mammifère en besoin d'un tel traitement d'une quantité thérapeutiquement efficace, dans laquelle ledit agoniste de récepteur D₄ est au moins 100 fois plus sélectif pour le récepteur D₄ que pour le récepteur D₂.

3. Utilisation selon la revendication 1 d'un agoniste de récepteur D₄ de la dopamine ou d'un sel pharmaceutiquement acceptable de celui-ci pour la fabrication d'un médicament destiné à traiter un dysfonctionnement sexuel féminin chez un mammifère par administration à un mammifère en besoin d'un tel traitement d'une quantité thérapeutiquement efficace, dans laquelle ledit agoniste de récepteur D₄ est au moins 100 fois plus sélectif pour le récepteur D₄ que pour le récepteur D₂.

4. Utilisation d'un agoniste de récepteur D₄ de la dopamine ou d'un sel pharmaceutiquement acceptable de celui-ci pour la fabrication d'un médicament destiné à traiter un dysfonctionnement érectile masculin chez un mammifère par administration à un mammifère en besoin d'un tel traitement d'une quantité thérapeutiquement efficace, dans laquelle ledit agoniste de récepteur D₄ est au moins 100 fois plus sélectif pour le récepteur D₄ que pour le récepteur D₂.

5. Utilisation selon l'une quelconque des revendications 1 à 4, dans laquelle ledit agoniste de récepteur D₄ de la dopamine sélectif est au moins 200 fois plus sélectif pour le récepteur D₄ que pour le récepteur D₂.

6. Utilisation selon l'une quelconque des revendications 1 à 4, dans laquelle ledit agoniste de récepteur D₄ de la dopamine sélectif est au moins 300 fois plus sélectif pour le récepteur D₄ que pour le récepteur D₂.

7. Utilisation selon l'une quelconque des revendications 1 à 4, dans laquelle ledit agoniste de récepteur D₄ de la dopamine sélectif est au moins 500 fois plus sélectif pour le récepteur D₄ que pour le récepteur D₂.

8. Utilisation selon l'une quelconque des revendications 1 à 4, dans laquelle ledit agoniste de récepteur D₄ de la dopamine sélectif est au moins 1000 fois plus sélectif pour le récepteur D₄ que pour le récepteur D₂.

9. Utilisation selon l'une quelconque des revendications 1 à 4, dans laquelle ledit agoniste de récepteur D₄ de la dopamine sélectif ne provoque pas de vomissement significatif.

10. Utilisation selon l'une quelconque des revendications 1 à 4, dans laquelle ledit agoniste de récepteur D₄ de la dopamine ou un sel pharmaceutiquement acceptable de celui-ci est en combinaison avec un véhicule pharmaceutiquement acceptable.

11. Utilisation selon la revendication 4 d'un ou de plusieurs agonistes de récepteur D₄ de la dopamine sélectifs ou d'un sel pharmaceutiquement acceptable de ceux-ci en combinaison avec un inhibiteur de la phosphodiestérase de type 5 ou d'un antagoniste d'un adrénorécepteur.

12. Utilisation selon l'une quelconque des revendications 2 à 4, dans laquelle ledit agoniste de récepteur D₄ de la dopamine sélectif est le N-{[4-(2-cyanophényl)-1-pipérazinyl]méthyl}-3-méthylbenzamide ou un sel pharmaceutiquement acceptable de celui-ci.

13. Utilisation selon l'une quelconque des revendications 2 à 4, dans laquelle ledit agoniste de récepteur D₄ de la dopamine sélectif est le 5-fluoro-2-{[4-(2-pyridinyl)-1-pipérazinyl]méthyl}-1H-indole ou un sel pharmaceutiquement acceptable de celui-ci.
